# FASCICULE DE BREVET EUROPEEN

(11) **EP 1 534 682 B1**
(45) Date de publication et mention de la délivrance du brevet: **27.12.2006**
(21) Numéro de dépôt: 03780203.0
(22) Date de dépôt: 29.07.2003
(51) Int. Cl.: C07D 221/18, A61K 8/49, A61Q 19/00, A61Q 19/06, A61Q 19/08, A61P 17/00

(54) **NOUVELLES MOLECULES DERIVEES DE LA NORAPORPHINE**
NEUE, VON NORAPORPHIN ABGELEITETE MOLEKÜLE
NOVEL MOLECULES DERIVED FROM NORAPORPHINE

(30) Priorité: 30.08.2002 FR 0210810
(43) Date de publication de la demande: 01.06.2005
(73) Titulaire: Sederma S.A.S., 78612 Le Perray-en-Yvelines Cedex (FR)
(72) Inventeur: LINTNER, Karl, F-78120 Rambouillet (FR)
(74) Mandataire: Laget, Jean-Loup
(86) Numéro de dépôt international: PCT/FR2003/002400
(87) Numéro de publication internationale: WO 2004/024695

(56) Documents cités:
- EP-A- 0 040 074
- EP-A- 0 848 041
- US-A- 3 131 191

## Description

La présente invention concerne des nouveaux composés dérivés de la noraporphine, les compositions cosmétiques et dermopharmaceutiques comprenant un ou plusieurs de ces composés, seuls ou en association avec un extrait de plante, notamment un extrait de *glaucium flavum,* et l'utilisation de ces compositions pour le soin du corps et de la peau, en particulier dans l'objectif de réduire l'hyperpigmentation, d'avoir un effet anti-âge, ou d'un amincissement.

La pigmentation naturelle de la peau relève d'un mécanisme aujourd'hui bien décrit : les mélanocytes présents dans la couche basale de l'épiderme produisent des pigments de mélanine, la synthèse s'effectuant au sein des mélanosomes. La synthèse de la mélanine (mélanogénèse) est augmentée sous l'action des UV. La fonction physiologique du bronzage qui s'ensuit est donc de protéger la peau contre les UV.

Divers dysfonctionnements du mécanisme de production de la mélanine (dus à un excès d'agressions extérieures, aux perturbations hormonales ou au vieillissement) provoquent l'apparition de taches brunes, notamment sous formes d'éphélides (taches de rousseur), de taches solaires ou de taches de sénescence.

Modifier la pigmentation naturelle de la peau est un souhait largement partagé par les femmes européennes, asiatiques, américaines, quoique à des titres divers : blancheur du teint, car trait de beauté pour les unes, ou atténuation des taches pigmentaires de sénescence révélatrices de l'âge pour les autres. En Asie comme en Europe et Amérique, la maîtrise de la pigmentation est donc un sujet sensible et l'objet d'une forte demande.

Trois enzymes clés sont impliquées dans la mélanogénèse : la tyrosinase et les tyrosine-related proteins (TRP-1 et TRP-2), toutes trois sont des glycoprotéines localisées dans la membrane des mélanosomes. Sur les trois, la tyrosinase est l'enzyme limitante en catalysant les deux premières étapes de la formation du pigment : ortho-hydroxylation de la tyrosine en L-DOPA puis oxydation de cette dernière en dopaquinone. TRP-1 et TRP-2 interviendraient en partie par une stabilisation de la tyrosine hydroxylase [PARK & GILCHREST, 1999].

D'autre part, la stimulation de la mélanogénèse passe par une augmentation des taux intracellulaires en AMPc, qui régulent à leur tour une activité protéine kinase PKC-b dont la capacité à phosphoryler la tyrosinase va être déterminante pour la synthèse mélanique [PARK & GILCHREST, 1999]. A l'appui de ce mécanisme on observera que l'irradiation UV augmente très significativement la PKC-b sur mélanocytes en culture.

Enfin, le rôle joué par le calcium intracellulaire dans le métabolisme du mélanocyte est sans doute aussi à prendre en compte.

Pour agir sur la pigmentation de la peau, il est donc possible d'envisager de dégrader la mélanine, de proposer des inhibiteurs de la mélanogénèse qui interagissent sur les diverses cibles décrites plus haut, voire d'inhiber la distribution de mélanine dans les couches de cellules épidermiques.

Cependant, la cible la plus couramment choisie est certainement la tyrosine hydroxylase puisqu'elle constitue une étape limitante du processus.

Longtemps, la dépigmentation ou l'éclaircissement de la peau ont été traités par des produits très puissants tels que l'hydroquinone, les composés phénoliques soufrés ou non, l'acide ascorbique, mais non dénués d'effets irréversibles d'hypopigmentation et d'irritation. Tous ces produits sont à utiliser dans un contexte efficacité / tolérance non adapté à la cosmétique.

En cosmétique, le problème a été abordé par l'utilisation des dérivés rétinoïdes, des AHA, de l'acide kojique, de l'arbutine. Les bons résultats obtenus *in vitro* sur cultures cellulaires sont rarement reproduits en utilisation *in vivo.*

L'hydroquinone, l'arbutine et l'acide kojique ont été développés pour l'inhibition compétitive de la tyrosinase [Arbutine, MAEDA 1996] ou inhibition de l'activité catalytique par chélation de l'ion cuivre indispensable à son fonctionnement [THIOLY et al., 1996]. Mais leur utilisation est délicate et peut entraîner des effets secondaires.

Des produits cosmétiques innovants, efficaces *in vivo,* et non toxiques sont donc fortement souhaités.

Augmenter le taux d'AMPc intracellulaire est aussi l'objectif des actifs amincissants. En effet l'AMPc intracellulaire est indispensable pour activer la libération de glycérol via la lipase (LHS) adipocytaire : par ce biais, il se produit un déstockage de matériel lipidique cellulaire, et donc, une diminution du volume de la cellule.

Après la génération des actifs amincissants basés sur une activation directe de la lipolyse via l'inhibition de la phosphodiestérase (caféine par exemple), on a vu l'émergence de produits plus sophistiqués s'adressant soit à la stimulation des récepteurs membranaires et à leurs systèmes de transduction intracellulaire (protéine G), soit à leur inhibition (récepteurs alpha et neuropeptide γ). Toutes ces approches visent à augmenter le taux d'AMPc intracellulaire.

Or il peut exister une voie alternative originale et même opposée aux système favorisant l'augmentation du pool intracellulaire d'AMPc dans le but de stimuler la lipolyse.

Le rôle central joué par le calcium intracellulaire dans le métabolisme du pré-adipocyte et de l'adipocyte mature est un phénomène bien documenté et il est clair que le Ca⁺⁺ intervient de plusieurs façons différentes dans l'installation de la masse graisseuse. Alors que celui-ci, par un flux entrant, inhibe la différentiation initiale des pré-adipocytes en diminuant le stockage des triglycérides, il joue un rôle contraire dans la phase terminale de différentiation ainsi que dans l'adipocyte mature en favorisant la lipogénèse. Pour comprendre ce phénomène il faut savoir qu'il existe une liaison structurale et fonctionnelle entre les sites membranaires de l'entrée calcique et l'adénylate cyclase.

En bloquant l'entrée du calcium, on favorise la phase initiale de différentiation car on lève alors l'inhibition de post-mitose calcium-dépendante, et on défavorise la phase terminale de différentiation en bloquant la lipogénèse.

Il est bien connu en Pharmacologie qu'un flux entrant de calcium favorisé par la norepinéphrine (agoniste α-adrénergique) peut être bloqué par des antagonistes α₁ tels que la prazosine et dans une moindre mesure par des antagonistes β₁-adrénergiques. On sait par ailleurs, qu'au sein d'une population adipocytaire, plus de 60 % des cellules expriment les récepteurs α₁ et β₁-adrénergiques.

Ce blocage par des antagonistes adrénergiques se traduit au niveau du pré-adipocyte et de l'adipocyte, par une diminution des marqueurs de différentiation que sont la glycerol-3-phosphate déshydrogénase (G-3-PDH) et le *"peroxisome proliferator-activated receptor gamma"* (PPARγ), ainsi que par la diminution du stockage des triglycérides.

Pour lutter efficacement contre les bourrelets et capitons, les consommatrices poussent l'industrie cosmétique au développement d'actifs de plus en plus performants.

Nous avons découvert de façon tout à fait surprenante que des molécules qui contiennent un noyau 1,2,9,10-tétrahydroxy-noraporphine dans leur structure possèdent à la fois un fort pouvoir d'inhibition de la mélanogénèse, un effet anti-oxydant, ainsi qu'une activité notable contre la lipogénèse.

L'invention faisant l'objet de cette demande réside dans le fait que nous avons découvert et démontré que les composés dérivés de la 1,2,9,10-tétrahydroxy-noraporphine de formule générale **I** réduisent la production de mélanine de façon efficace et non toxique, bloquent la lipogénèse, et présentent une activité anti-oxydante. Les nouveaux dérivés de la 1,2,9,10-tétrahydroxy-noraporphine objets de la présente demande présentent également un intérêt pour leur bonne biodisponibilité, solubilité, activité, stabilité, et profil toxicologique.

La présente invention concerne donc des compositions cosmétiques et dermopharmaceutiques comprenant un ou plusieurs composés de formule générale **I**, dérivés de la 1,2,9,10-tétrahydroxy-noraporphine : 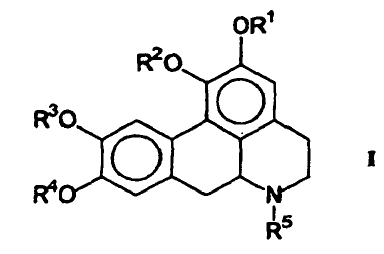 dans laquelle les groupes R¹, R², R³, R⁴ et R⁵, qui peuvent être identiques ou différents, représentent chacun un atome d'hydrogène, un groupe alkyle, aryle, aralkyle, acyle, sulfonyle, ou sucre.

Les composés de formule générale **I** selon l'invention peuvent exister sous leur forme libre, ou sous la forme d'un sel avec un acide cosmétiquement acceptable. La présente invention comprend aussi bien les formes libres ou sel de ces composés.

Par le terme « acide cosmétiquement acceptable » on entend au sens de la présente invention tout acide non toxique, y compris les acides organiques et inorganiques. De tels acides incluent les acides acétique, *p-*amino benzoïque, ascorbique, aspartique, benzènesulfonique, benzoïque, bis-méthylènesalicylique, bromhydrique, chlorhydrique, cinnamique, citraconique, citrique, éthanedisulfonique, fumarique, gluconique, glutamique, glyconique, itaconique, lactique, maléique, malique, mandélique, méthanesulfonique, mucique, nitrique, oxalique, palmitique, pamoïque, pantothénique, phosphorique, propionique, salicylique, stéarique, succinique, sulfamique, sulfurique, tartarique et paratoluènesulfonique. Les acides chlorhydrique et acétique sont particulièrement préférés.

Par le terme « groupe alkyle », on entend au sens de la présente invention tout groupe alkyle de 1 à 20 atomes de carbone linéaire ou ramifié, substitué ou non (notamment par un alcool, un acide carboxylique, une amine), saturé ou insaturé. En particulier un groupe alkyle est le groupe méthyle.

Par le terme « groupe aryle », on entend au sens de la présente invention un ou plusieurs cycles aromatiques ayant chacun de 5 à 8 atomes de carbone, pouvant être accolés ou fusionnés, substitués ou non. En particulier, les groupes aryles peuvent être des groupes phényle, ou naphtyle et les substituants des atomes d'halogène, des groupes alkoxy tels que définis ci-dessus, des groupes alkyles tels que définis ci-dessus ou le groupe nitro.

Par le terme « groupe aralkyle », on entend au sens de la présente invention tout groupe aryle tel que défini ci-dessus, lié par l'intermédiaire d'un groupe alkyle tel que défini ci-dessus. En particulier un groupe aralkyle est le groupe benzyle.

Par le terme « groupe acyle », on entend au sens de la présente invention tout groupe -C=OR⁶ où R⁶ peut être un groupe alkyle, aryle, aralkyle, amine, tels que définis ci-dessus. En particulier, un groupe acyle est le groupe acétyle (R⁶ = -CH₃).

Par le terme « groupe amine », on entend au sens de la présente invention tout groupe **-**NR⁷R⁸ où R⁷ et R⁸, qui peuvent être identiques ou différents, représentent chacun un atome d'hydrogène un groupe alkyle, aryle, aralkyle, acyle, sulfonyle, sucre, tels que définis ci-dessus.

Par le terme « groupe sulfonyle », on entend au sens de la présente invention tout groupe -SO₂R⁹ où R⁹ peut être un groupe alkyle, aryle, aralkyle, alkoxy, amine, tels que définis ci-dessus. En particulier, des groupe sulfonyles sont les groupes mésyle (R⁹ = -CH₃), triflyle (R⁹ = -CF₃), ou tosyle (R⁹ = -Ph-CH₃).

Par le terme « groupe alkoxy », on entend au sens de la présente invention tout groupe -OR¹⁰ où R¹⁰ peut être un groupe alkyle, aryle, aralkyle, acyle, sulfonyle, sucre, tels que définis ci-dessus.

Par le terme « groupe sucre » on entend au sens de la présente invention tout groupe hexose, oses et osides. En particulier, des groupes sucres sont les groupes glucose, arabinose, fructose, galactose, mannose, maltose, lactose, saccharose, cellobiose.

Les composés utilisés dans les compositions cosmétiques et/ou dermopharmaceutiques selon invention peuvent posséder un centre d'asymétrie et peuvent donc exister sous forme d'isomères optiques. La présente invention comprend aussi bien ces isomères soit séparément soit en tant que mélange.

Des composés particulièrement avantageux selon l'invention sont la 2,9-diacétyloxy-1,10-diméthoxy-6-méthyl-noraporphine (formule **II** = formule **I** où R¹ = R⁴ = -COCH₃, R² = R³ = CH₃, R⁵ = CH₃). 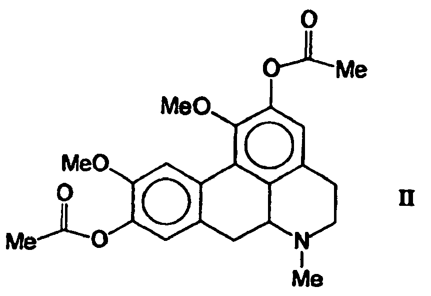 ainsi que la 1,2,9,10-tétraméthoxy-6-méthyl-noraporphine (formule I où R¹= R²=R³=R⁴=R⁵=CH₃).

Pour obtenir les effets décrits dans ce brevet, les composés de formule générale **I** utilisables dans les compositions cosmétiques et dermopharmaceutiques, peuvent être obtenus à partir de toute source d'approvisionnement, en particulier par voie de synthèse chimique, enzymatique, par l'une des nombreuses méthodologies de la biotechnologie, par extraction végétale ou par tout autre moyen utilisable permettant leur obtention à des coûts raisonnables dans le produit fini pour pouvoir être utilisés industriellement

Dans le cas d'une obtention d'origine végétale, il est évident que toute espèce végétale peut convenir à partir du moment où l'extrait, obtenu à partir d'une quelconque partie de l'organisme végétal, contient le ou les composés dérivant de la formule générale **I.** En particulier, une plante contenant des alcaloïdes de la famille de formule générale **I**, et en particulier la 1,2,9,10-tétraméthoxy-6-méthyl-noraporphine (formule I, R¹ = R² = R³ = R⁴= R⁵ = CH₃), est le *glaucium flavum.*

*Glaucium flavum,* de la famille des papaveraceae, est une plante européenne de taille moyenne (0,5 mètres environ), à la croissance tente et à fleurs hermaphrodites jaunes. Elle fleurit de juin à août, et ses graines mûrissent d'août à septembre.

Les solvants d'extraction peuvent être choisis parmi l'eau, le propylène glycol, le butylène glycol, la glycérine, le polyéthylène glycol, les éthers méthyliques et/ou éthyliques des diglycols, les polyols cycliques, les diglycols éthoxylés ou propoxylés, les alcools (méthanol, éthanol, propanol, butanol), ou tout mélange de ces solvants.

Par ailleurs, il est possible de réaliser des extraits de *glaucium flavum* par d'autres procédés comme, par exemple, la macération, la simple décoction, la lixiviation, l'extraction sous reflux, l'extraction supercritique, l'extraction au moyen d'ultrasons ou de micro-ondes ou enfin au moyen de techniques à contre courant, sans que cette liste ne soit limitative.

Certains dérivés de la noraporphine ont été synthétisés et décrits dans les brevets US-A-4 120 964 et US-A-4 202 980 en tant que substances agissant sur le système cardiovasculaire, pour le traitement de l'hypertension, administrées sous forme orale ou par injection. Ces substances n'ont jamais été décrites comme ingrédients actifs d'une quelconque composition cosmétique ou dermopharmaceutique.

La présente invention concerne donc également les compositions cosmétiques et dermopharmaceutiques contenant un ou plusieurs des composés ci-dessus de formule générale **I**, comprenant la 2,9-dihydroxy-1,10-diméthoxy-6-méthyl-noraporphine (formule **I**, R¹ = H, R² = R³ = CH₃, R⁴ = H, R⁵ = CH₃), la 1,2,10-triméthoxy-9-hydroxy-6-méthyl-noraporphine (formule **I**, R¹ = R² = R³ = CH₃, R⁴ = H, R⁵ = CH₃) et la 1,2,9,10-tétraméthoxy-6-méthyl-noraporphine (formule **I**, R¹ = R² = R³ = R⁴ = R⁵ = CH₃), seuls ou en association, et un excipient cosmétiquement acceptable. Ces trois dernières molécules, à savoir la 2,9-dihydroxy-1,10-diméthoxy-6-méthyl-noraporphine, la 1,2,10-triméthoxy-9-hydroxy-6-méthyl-noraporphine et la 1,2,9,10-tétraméthoxy-6-méthyl-noraporphine peuvent être obtenues soit par synthèse, soit par extraction végétale.

La ou les composés de formule générale **I** sont employés dans les compositions cosmétiques et dermopharmaceutiques conformes à l'invention à des concentrations qui peuvent varier entre 0,0001 % (p/p) et 50 % (p/p), préferentiellement entre 0,001 % (p/p) et 20 % (p/p).

Dans les compositions cosmétiques et dermopharmaceutiques, il peut être avantageux d'associer un ou des composés dérivés de la formule générale **I** avec un extrait de plante, et en particulier un extrait de *glaucium flavum* tel que défini ci-dessus.

L'extrait de *glaucium flavum* peut être utilisé soit sous forme liquide, soit sous forme sèche obtenue par précipitation, atomisation, évaporation ou lyophilisation. La quantité d'extrait de *glaucium flavum* à incorporer dans les préparations cosmétiques ou dermopharmaceutiques est comprise entre 0,01 et 100 % (p/p), préférentiellement entre 0,1 et 10 % en poids de la composition totale finale.

L'incorporation des extraits de *glaucium flavum* de quelque provenance que ce soit, dans les compositions cosmétiques est réalisée par tout type de procédé classiquement utilisé en cosmétologie et en dermopharmacie.

Les compositions sont par exemple des lotions, des laits ou des crèmes émollientes, des laits ou des crèmes pour les soins de la peau ou des cheveux, des crèmes, des lotions ou des laits démaquillants, des bases de fond de teint, des lotions, des laits ou des crèmes solaires, des lotions, des laits ou des crèmes de bronzage artificiel, des crèmes ou des mousses de rasage, des lotions après-rasage, des shampoings, des rouges à lèvres, des mascaras ou des vernis à ongles.

Ces compositions peuvent également se présenter sous la forme de bâtons pour les lèvres destinés soit à les colorer, soit à éviter les gerçures, ou de produits de maquillage pour les yeux ou des fards et fonds de teint pour le visage.

Lorsque les compositions selon l'invention se présentent sous la forme d'émulsions de type eau-dans-l'huile ou huile-dans-l'eau, la phase grasse est essentiellement constituée d'un mélange de corps gras d'extraction ou de synthèse, avec au moins une huile, et éventuellement un autre corps gras. La phase grasse des émulsions peut constituer de 5 à 60 % du poids total de l'émulsion.

La phase aqueuse desdites émulsions constitue de préférence de 30 à 85 % du poids total de l'émulsion. La proportion de l'agent émulsionnant peut être comprise entre 1 et 20 %, et de préférence entre 2 et 12 % du poids total de l'émulsion. Lorsque les compositions selon l'invention se présentent sous forme de lotions huileuses, oléoalcooliques ou hydroalcooliques, elles peuvent constituer, par exemple, des lotions antisolaires contenant un filtre absorbant les rayons UV, des lotions adoucissantes pour la peau ; les lotions huileuses peuvent en outre constituer des huiles moussantes contenant un tensioactif oléosoluble, des huiles pour le bain, etc.

Parmi les principaux adjuvants pouvant être présents dans les compositions selon l'invention, on peut citer les solvants organiques ou hydroglycoliques, y compris le MP-diol et les polyglycérines, les corps gras d'extraction ou de synthèse, les épaississants ioniques ou non ioniques, les adoucissants, opacifiants, stabilisants, émollients, les silicones, α- ou β-hydroxyacides, les agents anti-mousse, les agents hydratants, les vitamines, parfums, conservateurs, séquestrants, colorants, les polymères gélifiants et viscosants, les tensioactifs et émulsifiants, les autres principes actifs hydro- ou liposolubles, les extraits végétaux, extraits tissulaires, extraits marins, filtres solaires, les antioxydants.

Les mono-ou poly-alcools plus particulièrement préférés sont choisis parmi l'éthanol, l'isopropanol, le propylène-glycol, le glycérol et le sorbitol.

A titre de corps gras, parmi les huiles minérales, on peut citer l'huile de vaseline ; parmi les huiles animales, les huiles de baleine, de requin, de phoque, de menhaden, de foie de flétan, de morue, de thon, de tortue, de pied de boeuf, de pied de cheval, de pied de mouton, de vison, de loutre, de marmotte, etc. ; parmi les huiles végétales, les huiles d'amande, de germes de blé, de jojoba, de sésame, de tournesol, de palme, de noix, de karité, de shoréa, de macadamia, de pépins de cassis et similaires.

Parmi les esters d'acides gras, on peut utiliser des esters d'acide en C₁₂ à C₂₂ saturés ou insaturés et d'alcools inférieurs comme l'isopropanol ou le glycérol ou d'alcools gras en C₈ à C₂₂, linéaires ou ramifiés, saturés ou insaturés ou encore d'alcanediols-1,2 en C₁₀-C₂₂.

On peut également citer comme corps gras, la vaseline, la paraffine, la lanoline, la lanoline hydrogénée, le suif, la lanoline acétylée, les huiles de silicone.

Parmi les cires, on peut citer la cire de Sipol, la cire de lanoline, la cire d'abeille, la cire de Candelila, la cire monocristalline, la cire de Carnauba, le spermaceti, le beurre de cacao, le beurre de karité, les cires de silicone, les huiles hydrogénées concrètes à 25°C, les sucroglycérides, les oléates, myristates, linoléates et stéarates de calcium, magnésium et aluminium.

Parmi les alcools gras, on peut citer les alcools laurique, cétylique, myristique, stéarique, palmitique, oléique, et les alcools de GUERBET comme le 2-décyltétradécanol ou le 2-hexyldécanol. A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de glycérol, les alcools en C₁₂-C₁₈ comportant de 2 à 10 moles de glycérol. Il peut être aussi utile d'utiliser des épaississants tels que les dérivés de cellulose, les dérivés de d'acide polyacrylique, les gommes de guar ou de caroube ou la gomme de xanthane.

Les compositions selon l'invention peuvent inclure d'autres ingrédients variés et additionnels, conventionnels ou non. Bien sûr la décision d'inclure un ingrédient et le choix d'un actif spécifique et des ingrédients additionnels dépend de l'application spécifique et de la formulation du produit. La ligne de démarcation entre un ingrédient "actif' et un ingrédient "additionnel" est donc artificielle et dépend de l'application spécifique et du type de produit. Une substance qui est un ingrédient "actif" dans une application ou un produit peut être un ingrédient "additionnel" ou "fonctionnel" dans une autre, et *vice versa.*

Les compositions selon la présente invention peuvent donc inclure un ou plusieurs ingrédients actifs, qui apporteront un certain avantage à l'objet de l'application de la composition, par exemple la peau ou les cheveux. Ces ingrédients actifs peuvent inclure une ou plusieurs substances comme des agents de nettoyage, agents de soin capillaire, agent de soin pour la peau, agent de coiffage, agent antipelliculaire, agent de repousse de cheveux, parfums, écrans solaires, pigments, hydratants, agents filmogènes, colorants capillaires, maquillages, détergents, agents épaississants, émulsifiants, agents antiseptiques, actifs déodorants et surfactants, propulseurs.

Le choix de l'actif ou des actifs dépend de la nature du produit cosmétique ou de soin à formuler. Par exemple, les filtres solaires peuvent être utilisés dans des lotions anti-solaires, dans des shampooings, dans des lotions de soin capillaire, et ainsi de suite. Pour chaque type d'actif, un ou plusieurs ingrédients peuvent être présents. De même, plus d'un type d'actif peut être présent dans une composition.

Le CTFA Cosmetic ingrédient Handbook, Ninth Edition (2002) liste beaucoup des ingrédients cosmétiques habituellement utilisés dans l'industrie cosmétique, qui conviennent pour être utilisés dans les compositions selon la présente invention. Des exemples de classes d'ingrédients incluent : les abrasifs, les absorbants, les huiles essentielles, les astringents, les agents anti-agglomérants, les agents anti-mousses, les agents anti-microbiens, les antioxidants, les liants, les additifs bilologiques, les tampons, les agents de volume, les agents chelatants, les additifs chimiques, les colorants, les astringents cosmétiques, les biocides cosmétiques, les dénaturants, les astringents de médicaments, les analgésiques externes, les agents filmogènes, les agents opacifiants, les ajusteurs de pH, les propellants, les agents de réduction, les séquestrants, les agents de soin de la peau (par exemple les humectants), les agents de traitement de la peau, les épaississants, mais aussi les actifs pris parmi te groupe consistants des actifs de desquamation, les actifs anti-acné, la vitamine C et ses dérivés, les vitamines B1 à B12 leurs dérivés, la vitamine E et ses dérivés, la vitamine H, la vitamine K, la vitamine A et les retinoïdes, les peptides, les hydroxy acides, les anti-oxidants, les piégeurs de radicaux, les chélatants, les actifs anti-inflammatoires, les actifs bronzants, les actifs éclaircissants de la peau, actifs anti-cellulite, les actifs anti-microbiens, les actifs anti-rides, les actifs stimulant la lipolyse, les actifs stimulant la lipogénèse, les agents anti-stress, les inhibiteurs de la protéolyse, en particulier les inhibiteurs de la MMP, les enzymes, les ceramides et leurs analogues, les anti-irritants et les actifs adoucissant la peau, les actifs anti-pollution, les actifs cicatrisants, les hydratants, les émollients, les actifs de protection anti-solaire, les écrants solaires et filtres UV, les actifs raffermissants, les liposomes.

En particulier, les ingrédients de soin capillaires qui peuvent être combinés dans les compositions selon la présente invention peuvent être trouvés sur www.sederma.fr, ou dans les paragraphes qui suivent.

Les compositions selon la présente invention peuvent également contenir une quantité suffisante d'actifs anti-acné. Des exemples d'actifs anti-acné incluent la composition appelée ac.net® (commercialisé par SEDERMA, France) et ses composants individuels (acide nordihydroguaiarétique, acide oléanolique), et aussi le résorcinol, le soufre, l'acide salicylique, le peroxyde de benzoyle, l'érythromycine, le zinc, etc. D'autres exemples d'actifs anti-acné sont décrits en détails dans le brevet U.S. Pat. No. 5 607 980, délivré à McAtee *et al.,* le 4 mars 1997.

Les compositions selon la présente invention peuvent également contenir une quantité suffisante d'un ou plusieurs actifs anti-rides. Des exemples d'actifs anti-rides convenant à l'utilisation dans les compositions selon la présente invention incluent les alpha-hydroxy acides comme l'acide lactique et l'acide glycolique ou les béta-hydroxy acides comme l'acide salicylique et ses dérivés, les vitamines, en particulier la vitamine 83 et tous les rétinoïdes. Les isoflavones et les phytostérols conviennent aussi particulièrement.

Des peptides, et notamment des di-, tri-, tetra-, et pentapeptides et leurs dérivés peuvent être inclus dans les compositions selon la présente invention en quantité suffisante. Les peptides peuvent être naturels ou synthétiques. Les dipeptides peuvent être, sans être limités à cette liste, Tyr-Arg, Val-Trp, Asn-Phe, Asp-Phe, beta-Ala-His (Camosine), N-palmitoyl-beta-Ala-His, Tyr-Arg-hexadecylester, et leurs dérivés. Les tripeptides incluent Gly-His-Lys, Arg-Lys-Arg, His-Gly-Gly, Lys-Phe-Lys, Lys-Phe-Lys et leurs analogues de substitution conservative, Gly-His-Lys, Gly-Lys-His, Arg-Lys-Arg-NH₂, et leurs dérivés. Les tetrapeptides incluent Gly-Gln-Pro-Arg (Rigin), Thr-Lys-Pro-Arg (Tuftsin) Lys-Asn-Pro-Tyr, Lys-Asn-Gly-Tyr, Lys-Asn-(D-Pro)-Tyr, Lys-Asn-Pro-Phe, (D-Lys)-Asn-Pro-Tyr, Lys-Gln-Pro-Tyr, Gly-Asn-Pro-(D-Arg), Gly-Asn-Pro-Tyr, (D-Lys)-Asn-Gly-Tyr, (D-Lys)-Gln-Pro-Tyr and (D-Lys)-Asn-Pro-Phe et leurs dérivés et analogues par substitution conservative. Les pentapeptides et hexapeptides peuvent être, sans être limités à cette liste, Lys-Thr-Thr-Lys-Ser, Tyr-Gly-Gly-Phe-X avec X = Met or Leu ou des mélanges, Val-Gly-Val-Ala-Pro-Gly et leurs dérivés. Ces peptides seront utilisés sous leurs formes libres ou N-acylées. En particulier, un dipeptide préféré est le N-Ac-Tyr-Arg-hexadecylester (CALMOSENSINE® de SEDERMA, France). Un tripeptide préféré est le N-palmitoyl-Gly-His-Lys (BIOPEPTIDE CL de SEDERMA, France), le Peptide CK (Arg-Lys-Arg) et la Lipospondine (N-elaidoyl-Lys-Phe-Lys) et ses analogues de substitution conservative, le Peptide CK+ (N-Ac-Arg-Lys-Arg-NH₂). Un tetrapeptide préféré est le N-palmitoyl-Gly-Gln-Pro-Arg et un pentapeptide préféré est le N-Pat-Lys-Thr-Thr-Lys-Ser, disponible sous le nom MATRIXYL® de SEDERMA, France.

Les compositions selon la présente invention peuvent inclure des antioxydants et/ou des piégeurs de radicaux pour protéger la peau des dommages causés par une exposition aux UV. Des anti-oxydants/piégeurs de radicaux comme l'acide ascorbique (vitamine C) et ses sels, les esters ascorbiques des acides gras, les dérivés de l'acide ascorbique (par exmple magnésium ascorbyl phosphate, sodium ascorbyl phosphate, ascorbyl sorbate), le tocopherol (vitamine E), le sorbate de tocopherol, l'acétate de tocopherol, les autres esters du tocopherol, les acides benzoïques hydroxy dutylés et leurs sels, 6-hydroxy acide (disponible sous le nom TROLOX®), l'acide gallique et ses alkyls esters, et notamment le propyl gallate, l'acide urique et ses sels et ses alkyl esters, l'acide sorbique et ses sels, l'acide lipoïque, les amines (par exemple la N, N-diéthylhydroxylamine, l'amino-guanidine), les composés sulfhydryles (par exemple glutathion), l'acide dihydroxy fumarique et ses sels, la lysine pidolate, l'arginine pidolate, l'acide nordihydroguaiaretique, les bioflavonoides, le curcumin, la lysine, la methionine, la proline, la superoxide dismutase, les Extremozymes comme celle proposée sous le nom VENUCEANE® (commecialisée par SEDERMA, France), la silymarine, les extraits de thé, les extraits de peau/grains de raisin, la mélanine, et les extraits de romarin peuvent être utilisés.

Les flavonoïdes convenant à la présente invention sont les flavanones choisies parmi les flavanones non substituées, les flavanones mono-substitutées, et leurs mélanges ; les chalcones sélectionnées parmi les chalcones non-substituées, les chalcones mono-substituées, les chalcones di-substituées, les chalcones tri-substituées, et leurs mélanges ; les flavones choisies parmi les flavones non-substituées, les flavones mono-substituées, les flavones di-substituées, et leurs mélanges ; une ou plusieurs isoflavones ; les coumarines choisies parmi les coumarines non-substituées, les coumarines mono-substituées, les coumarines di-substituées, et leurs mélanges ; les chromones choisies parmi les chromones non-substituées, les chromones mono-substituées, les chromones di-substituées, et leurs mélanges ; un ou plusieurs dicoumarols ; une ou plusieurs chromanones ; un ou plusieurs chromanols; leurs isomères (par exemple les isomères cis/trans); et leurs mélanges.

D'autres exemples de flavonoïdes peuvent être trouvés dans la demande PCT No. WO 00/62743 déposée par Larry R. Robinson *et al.* le 19 avril, 2000, publiée le 26 Octobre, 2000. Ils peuvent être obtenus comme extraits de sources naturelles (plantes, algues), comme produits d'hémisynthèse ou de synthèse. Des mélanges des composés flavonoïdes ci-dessus peuvent aussi être utilisés.

Les compositions selon la présente invention peuvent contenir un agent éclaircissant de la peau. Les agents éclaircissants de la peau incluent l'acide kojique, l'arbutine, l'acide ascorbique et ses dérivés (notamment magnésium ascorbyl phosphate ou sodium ascorbyl phosphate), et les extraits (notamment extrait de citrus unshiu, les extraits de busserole et mitracarpus, respectivement disponibles comme MELASLOW®, LUMISKIN® et ETIOLINE® de SEDERMA, France).

Les agents anti-inflammatoires peuvent être incorporés dans des compositions selon la présente invention, comme des extraits naturels de plantes, fungi, algues. Par exemple, l'acide ursolique, l'acide nordihydroguaiaretique, l'extrait de kava-kava, l'extrait de bacopa monnieri (BACOCALMINE® de SEDERMA, France), la cire de candelilla, le bisabolol, l'aloe vera, les stérols de plantes, la camomille, l'extrait de trèfle rouge (commecrialisé sous le nom STEROCARE® de SEDERMA, France), et les extraits de sea whip, peuvent être utilisés. D'autres agents anti-inflammatoires utiles incluent les composés de la famille de la réglisse (du genre/espèce Glycyrrhiza glabra), incluant l'acide glycyrrhétique, l'acide glycyrrhizique, et leurs dérivés (notamment leurs sels et esters).

Lorsque les compositions selon l'invention sont des dispersions, il peut s'agir de dispersions de lécithine dans l'eau en présence de tensioactif ou encore de dispersions aqueuses de sphérules lipidiques, constituées de couches moléculaires organisées enfermant une phase aqueuse encapsulée. On peut citer, à cet effet, comme composés lipidiques, les alcools et diols à longue chaîne, les stérols tels que le cholestérol, les phospholipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras tels que le dicétylphosphate acide ou son sel de sodium, les alkylsulfates tels que le cétylsulfate de sodium, les acides gras sous forme de sels ou encore les lipides du type de ceux décrits dans les brevets français n° 2 315 991, 1 477 048 et 2 091 516 ou dans les demandes de brevet international WO 83/01 571 et WO 92/08 685.

On peut par exemple utiliser comme autres lipides, des lipides comportant une chaîne lipophile longue contenant 12 à 30 atomes de carbone, saturée ou insaturée, ramifiée ou linéaire, par exemple une chaîne oléique, lanolique, tétradécylique, hexadécylique, isostéarylique, laurique ou alcoylphénylique. Le groupement hydrophile de ces lipides peut être un groupement ionique ou non-ionique. A titre de groupements non-ioniques, on peut citer des groupements dérivés de polyéthylèneglycol. On peut aussi utiliser avantageusement comme lipides formant la phase lamellaire, des éthers de polyglycérol tel que ceux décrits dans les brevets français n° 1 477 048 , 2 091 516, 2 465 780 et 2 482 128.

A titre de groupement ionique, on peut avantageusement utiliser un groupement dérivé d'un composé amphotère, anionique ou cationique.

D'autres lipides décrits dans la demande de brevet international WO 83/01 571 comme pouvant être utilisés pour la formation de vésicules sont les glycolipides comme le lactosylcéramide, le galactocérébroside, les gangliosides et le trihexosylcéramide, ainsi que les phospholipides tels que le phosphatidylglycérol et le phosphatidylinositol.

Les substances actives peuvent être des substances ayant un intérêt pharmaceutique, alimentaire ou ayant une activité cosmétique. Lorsqu'elles sont hydrosolubles, elles peuvent être dissoutes de façon homogène ou elles sont dans la phase aqueuse encapsulée à l'intérieur des vésicules. Les substances hydrosolubles ayant une activité cosmétique et/ou pharmaceutique peuvent être des produits destinés aux soins ou aux traitements de la peau et du cheveu tels que par exemple des humectants comme la glycérine, le sorbitol, le pentaérythritol, l'acide pyrrolidone carboxylique et ses sels ; des agents de brunissage artificiel tels que la dihydroxyacétone, l'érythrulose, le glycéraldéhyde, les γ-dialdéhydes tels que l'aldéhyde tartique, ces composés étant éventuellement associés à des colorants ; des filtres solaires hydrosolubles; des antiperspirants, des déodorants, des astringents, des produits rafraîchissants, toniques, cicatrisants, kératolytiques, dépilatoires, des eaux parfumées ; des extraits de tissus végétaux, tels que les polysaccharides ; des colorants hydrosolubles ; des agents antipelliculaires ; des agents antiséborrhéiques, des oxydants tels que des agents de décoloration comme l'eau oxygénée ; des réducteurs tels que l'acide thioglycolique et ses sels.

On peut citer également les vitamines, les hormones, les enzymes, telles que la superoxyde dismutase, les vaccins, les anti-inflammatoires tels que l'hydrocortisone, les antibiotiques, les bactéricides, les agents cytotoxiques ou anti-tumoraux.

Lorsque les substances actives sont liposolubles, elles peuvent se trouver incorporées dans les feuillets des vésicules. Elles peuvent être choisies dans le groupe formé par les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

Les composés de formule générale **I,** ainsi que les compositions cosmétiques et dermopharmaceutiques les contenant seuls ou en association, peuvent être utilisés dans les compositions cosmétiques conformes à l'invention soit en ajout individuel, soit en tant que pré-mélange dans un excipient convenable, et utilisés sous forme de solution, de dispersion, d'émulsion, de pâte ou de poudre. Ils peuvent individuellement ou ensemble être véhiculés par des vecteurs cosmétiques tels que les macro-, micro- ou nanocapsules, des liposomes ou des chylomicrons, les macro-, micro- ou nanoparticules ou microéponges. Ils peuvent être également adsorbés sur des polymères organiques poudreux, les talcs, bentonites et autres supports minéraux.

Les composés de formule générale **I**, ainsi que les compositions cosmétiques et dermopharmaceutiques les contenant seuls ou en association peuvent être utilisés dans une forme quelconque, ou sous une forme liée ou incorporée ou absorbée ou adsorbée à de macro-, micro- et nanoparticules, de macro-, micro- et nanocapsules, pour le traitement des textiles, fibres synthétiques ou naturelles, laines et tous matériaux susceptibles d'être utilisés pour réaliser des vêtements et sous-vêtements de jour ou de nuit destinés à être en contact avec la peau tel que les collants, sous-vêtements, mouchoirs, lingettes, pour exercer leur effet cosmétique via ce contact textile/peau et permettre une délivrance topique continue.

La présente invention concerne également l'utilisation d'un ou plusieurs de ces composés de formule générale **I**, et en particulier la 2,9-diacétyloxy-1,10-diméthoxy-6-méthyl-noraporphine (formule I où R¹= R⁴=COCH₃, R² = R³ = CH₃, R⁵ = CH₃) ou la 1,2,9,10-tétraméthoxy-6-méthyl-noraporphine (formule I, R¹ = R² = R³ = R⁴ = R⁵ = CH₃), et l'utilisation des compositions cosmétiques et dermopharmaceutiques contenant un ou plusieurs de ces composés, seuls ou en association avec un extrait de plante, notamment le *glaucium flavum,* comme ou pour la fabrication de compositions cosmétiques ou dermopharmaceutiques dans l'objectif d'une diminution de la pigmentation, en particulier pour l'éclaircissement du teint, l'atténuation des taches cutanées de sénescence, l'homogénéisation de la coloration de la peau, l'éclaircissement de toute pigmentation associée à la mélanine, y compris celle des cheveux, ou dans l'objectif d'un traitement anti-âge, anti-vieillissement, antiradicalaire, antioxydant, ou encore dans l'objectif d'un amincissement, d'une réduction de la peau d'orange et/ou de la surcharge pondérale des hanches et des cuisses, du traitement de la cellulite, du raffermissement cutané, d'un amincissement de certaines parties du visage, plus généralement de l'inhibition de la lipogénèse.

Ces composés de formule générale **I**, ainsi que les compositions cosmétiques et dermopharmaceutiques les contenant seuls ou en association peuvent également être utilisées pour la préparation de médicaments destinés aux soins de la peau, particulièrement à son blanchissement et à sa moindre coloration lors d'exposition aux UV naturels ou artificiels, ou dans l'objectif d'un traitement and-âge, anti-vieillissement, antiradicalaire, antioxydant, ou encore dans l'objectif d'un traitement amincissant, d'une réduction de la peau d'orange et/ou de la surcharge pondérale des hanches et des cuisses, le traitement de la cellulite et le raffermissement cutané, plus généralement de l'inhibition de la lipogénèse.

En outre, les substances et compositions objets du présent brevet peuvent être utilisées pour fabriquer des tissus, textiles et vêtements à effet cosmétique, notamment pour éclaircir la peau ou les cheveux, ou agir contre les effets du temps, ou pour amincir, réduire la peau d'orange.

A titre d'illustration, suivent des exemples, non limitatifs, de la mise en oeuvre de la présente invention.

### Exemple n°1 : Synthèse de la 2,9-diacétyloxy-1,10-diméthoxy-6-méthyl-noraporphine (composé II)

A une solution de 2,9-dihydroxy-1,10-diméthoxy-6-méthyl-noraporphine (1,01 g ; 3,09 mmoles) dans 20 ml de dichlorométhane (DCM) sont successivement additionnés, à température ambiante, 3,09 équivalents d'anhydride acétique (Ac₂O) (900 µl, 9,52 mmoles), puis 1,99 équivalents de diisopropyléthylamine (DIEA) (1,05 ml, 6,13 mmoles). Après une nuit d'agitation à température ambiante à l'abri de la lumière, de l'éther de pétrole (50 ml) et de l'eau (50 ml) sont ajoutés. Après extraction, la phase organique est séchée sur sulfate de sodium anhydre (5 g), filtrée sur fritté et évaporée. 1,15 g (2,795 mmoles ; 90,5 %) de 2,9-diacétyloxy-1,10-diméthoxy-6-méthyl-noraporphine sont isolés sous la forme d'un solide jaune inodore après une nuit de séchage au dessiccateur.
C₂₃H₂₅NO₆
MM = 411,4588 gmol⁻¹
Point de Fusion : 80-81 °C

| | | | | |
|---|---|---|---|---|
| CHN: | Calculé: | 67,14 % C ; | 6,12 % H ; | 3,40 % N |
| | Trouvé : | 67,26 % C ; | 6,07 % H ; | 3,38 % N |

Infra Rouge : 2955 ; 2895 ; 2835 ; 2789 ; 1766 ; 1695 ; 1515 ; 1462 ; 1421 ; 1368 ; 1316 ; 1199 ; 1079 ; 1000 ; 906 cm⁻¹.
Spectrométrie de masse : (m/z) = 412,4 [M+H]⁺

| **Exemple n°2** : Crème de jour | g/100 g |
|---|---|
| Volpo S20 | 2,4 |
| Volpo S2 | 2,6 |
| Prostéaryl 15 | 8,0 |
| Cire d'abeille | 0,5 |
| Stéaroxy diméthicone | 3,0 |
| Propylène glycol | 3,0 |
| Carbomer | 0,25 |
| Triéthanolamine | 0,25 |
| 2,9-diacétyloxy-1,10-diméthoxy-6-méthyl-noraporphine | 2,5.10⁻³ |
| Eau, conservateurs, parfum | qsp 100 g |

Cette émulsion est utilisée pour éclaircir et hydrater la peau du visage.

| **Exemple n°3 : Gel amincissant** | **g/100 g** |
|---|---|
| Carbopol® 1342 | 0,3 |
| Propylène glycol | 2,0 |
| Glycérine | 1,0 |
| Vaseline blanche | 1,5 |
| Cylomethicone | 6,0 |
| Alcool cétylique | 0,5 |
| Lubrajel® MS | 10,0 |
| Triéthanolamine | 0,3 |
| 2,9-diacétyloxy-1,10-diméthoxy-6-méthyl-noraporphine | 0,01 |
| Eau, conservateurs, parfum | qsp 100 g |

| **Exemple n°4 : Crème de massage amincissante** | **g/100g** |
|---|---|
| Ultrez 10 | 0,20 |
| Butylène glycol | 5,0 |
| Acide stéarique | 1,50 |
| Crodamol GTCC | 2,00 |
| Petrolatum oil | 2,00 |
| Crodacol C90 | 0,50 |
| Crodafos CES | 1,50 |
| Extrait de *glaucium flavum* | 3,00 |
| 1,2,9,10-tétraméthoxy-6-méthyl-noraporphine | 0,02 |
| eau, conservateurs, parfum | qsp 100 g |

### Exemple N°5 : Inhibition de la synthèse de mélanine (in vitro)

L'efficacité des produits sur la mélanisation, a été testée en culture de mélanocytes humains normaux (MHN). Ce milieu de culture est classiquement utilisé pour tester les variations de taux de mélanine. Les cellules sont incubées en présence du produit à tester pendant 7 jours, les cellules témoins sont incubées en milieu de culture seul.

Après 7 jours, la mélanine totale (phaéomélanine et eumélanine) présente dans les cellules est obtenue après lyse des cellules et solubilisation par la soude, le dosage est colorimétrique [adaptation de la méthode de MAEDA K., FUKUDA M., 1991].

Les taux de mélanine produits en présence du produit à tester à différentes concentrations sont comparés à ceux obtenus dans les cellules témoins. Les données sont normalisées par la teneur en protéine de l'échantillon.

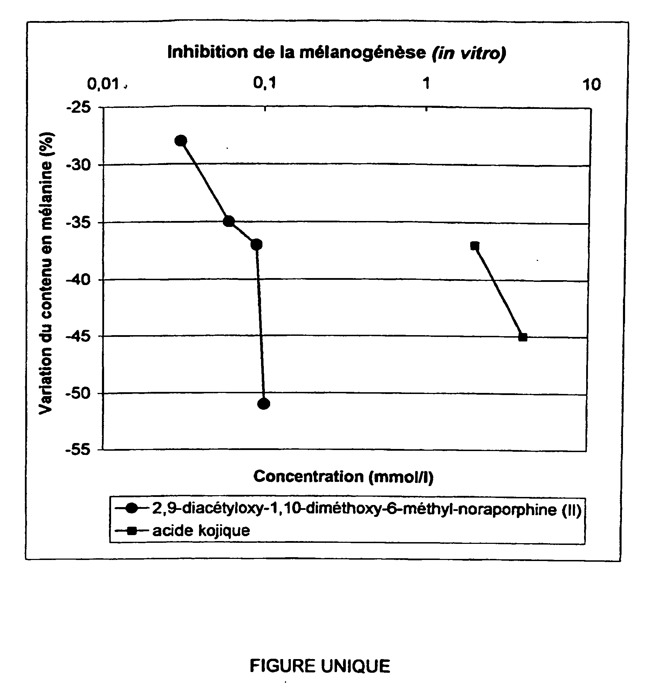

La figure unique montre la variation de synthèse de la mélanine sous l'effet d'un contact de 7 jours avec l'acide kojique (témoin positif) d'une part, et la 2,9-diacétyloxy-1,10-diméthoxy-6-méthyl-noraporphine (composé **II**) faisant l'objet du présent brevet d'autre part. L'inhibition de synthèse observée en 7 jours de contact pour ces produits est dépendante de la concentration testée. Cette inhibition varie de - 28 % à - 51 %. Nous montrons ainsi que ce composé a une activité d'inhibition de la mélanogénèse tout à fait intéressante.

### Exemple 6 : Inhibition de la lipogénèse (in vitro)

Le test suivant est basé sur le fait que des fibroblastes 3T3 L1, en culture, ont la particularité de se différencier sous l'action d'un cocktail de substances (messagers hormonaux), en pré-adipocytes puis en adipocytes chargés de lipides.

La culture se déroule en trois étapes : multiplication cellulaire jusqu'à confluence, ajout du cocktail de différentiation (étape au cours de laquelle on obtient les pré-adipocytes initiaux (72 heures)), puis différentiation active avec stimulation de la lipogénèse (environ 72 heures) à la fin de laquelle le stockage apparent en gouttelettes lipidiques est alors nettement visible au microscope.

**L'enzyme G-3-PDH (Glycérol-3-Phosphate déshydrogénase),** indispensable à la synthèse de triglycérides est exprimée très fortement pendant cette étape active de stockage lipidique.

Le produit à tester est ajouté à la deuxième étape.

A l'issue de la période d'incubation on compare l'activité G-3-PDH entre les pré-adipocytes témoins et ceux incubés en présence du produit testé.

Dans ces conditions, un produit qui inhibe la lipogénèse provoque une baisse de l'activité G-3-PDH.

### Protocole suivi

Après la différentiation provoquée, on ajoute à la culture des pré-adipocytes la solution à tester, ici une solution de 2,9-diacétytoxy-1,10-**diméthoxy-6-mëthyl-noraporphine** (composé **II).** Deux cultures témoins, l'une en témoin négatif et l'autre en témoin positif sont menées en parallèle.

En fin d'incubation, les cellules sont prélevées et lysées et le test est effectué sur le contenu intracellulaire.

L'activité G-3-PDH est mesurée par disparition du NADH (λ= 340 nm).

### Inhibition de l'activité G-3-PDH :

Le tableau suivant montre les moyennes des mesures (inhibition - en % du témoin - de l'activité G-3-PDH en culture de pré-adipocytes traitée par la 2,9-diacétyloxy-1,10-diméthoxy-6-méthyl-noraporphine, composé **II)** réalisées sur 3 essais indépendants les uns des autres. Les valeurs de l'activité enzymatique sont normalisées au nombre de cellules.

| | **ACTIVITE G-3-PDH / 10⁶ cellules** |
|---|---|
| **II - 0,03 mmol/l** | **- 49 %** |
| **II - 0,06 mmol/l** | **- 67 %** |
| **II - 0,09 mmol/l** | **- 76 %** |

Ces résultats démontrent clairement l'effet de la 2,9-diacétyloxy-1,10-diméthoxy-6-méthyl-noraporphine (composé **II**) sur l'activité G-3-PDH, puisqu'une inhibition de la G-3-PDH de 76 % environ est obtenue en présence de seulement 0,09 mmol/l du composé **II.** De plus, cet effet est incontestablement concentration-dépendant.

L'inhibition très importante de la G-3-PDH démontre le pouvoir inhibiteur de la 2,9-diacétyloxy-1,10-diméthoxy-6-méthyl-noraporphine (composé **II**) sur la lipogénèse dans les préadipocytes.

### Morphologie des préadipocytes :

La morphologie des cellules au microscope montre une population adipocytaire avec peu d'inclusions lipidiques par rapport aux cellules témoins.

### Exemple 7: Inhibition de la peroxydation (in vitro)

Afin de rechercher une activité anti-oxydante, nous avons évalué l'effet de la 2,9-diacétyloxy-1,10-diméthoxy-6-méthyl-noraporphine (composé **II)** sur l'inhibition de la peroxydation induite sur des liposomes par des UVA ou le couple H₂O₂ /FeCl₂.

### UVA:

Les liposomes, une fois fabriqués, reçoivent les produits à tester en solution, puis sont mis sous la lampe UVA et sont irradiés à 10 J/cm². Les liposomes sont ensuite mis à incuber à 45°C. Après 24 heures, la peroxydation lipidique est dosée en estimant le taux de marqueur diènes conjugués. L'effet du produit est comparé au contrôle négatif.

| | Inhibition perox. UVA |
|---|---|
| **II -** 0,03 mmol/l | - 93 % |
| **II -** 0,06 mmol/l | - 87 % |
| **II -** 0,09 mmol/l | - 89 % |
| **II-**0.15 mmol/l | ≈- 100 % |
| **II -** 0,30 mmol/l | ≈- 100 % |

Aux concentrations testées, la 2,9-diacétyloxy-1,10-diméthoxy-6-méthyl-noraporphine (composé **II)** inhibe presque totalement la peroxydation lipidique UVA induite.

### H₂O₂/FeCl₂ :

Une forte inhibition (- 60 % à 0,06 mmol/l) est également obtenue dans un protocole similaire quand on ajoute le couple H₂O₂ / FeCl₂ (réaction de FENTON) à la solution de liposomes.

## Revendications

1. Compositions cosmétiques et dermopharmaceutiques comprenant un ou plusieurs composés répondant à la formule I : 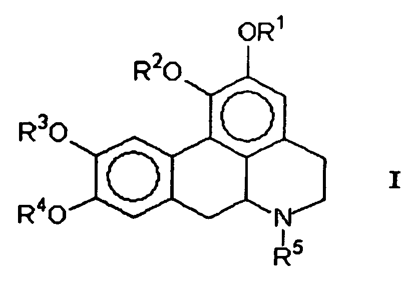 dans laquelle :
les groupes R¹, R², R³, R⁴ et R⁵, qui peuvent être identiques ou différents, représentent chacun un atome d'hydrogène, un groupe alkyle, aryle, aralkyle, acyle, sulfonyle, ou sucre,
et leur sel avec un acide cosmétiquement acceptable, sous forme de leur isomère optiquement pur ou d'un mélange d'isomères,
seuls ou en association, et un excipient cosmétiquement acceptable.

2. Compositions selon la revendication 1 **caractérisées en ce que** le composé est la 2,9-diacétyloxy-1,10-diméthoxy-6-méthyl-noraporphine (formule **I** où R¹ = R⁴ = COCH₃, R² = R³ = CH₃, R⁵ = CH₃) ou la 1,2,9,10-tétraméthoxy-6-méthyl-noraporphine (formule I où R¹ = R² = R³ = R⁴ = R⁵ = CH₃).

3. Compositions selon la revendication 1 ou 2, **caractérisées en ce que** les composés sont obtenus à partir de toute source d'approvisionnement, en particulier par voie de synthèse chimique, ou par extraction végétale.

4. Compositions selon la revendication 3, **caractérisées en ce que** les composés sont obtenus par extraction végétale à partir de *glaucium flavum.*

5. Compositions cosmétiques ou dermopharmaceutiques selon l'une quelconque des revendications 1 à 4, **caractérisées en ce que** les composés sont utilisés à des concentrations variant entre 0,0001 % (p/p) et 50 % (p/p), préférentiellement entre 0,001 % (p/p) et 20 % (p/p).

6. Compositions cosmétiques ou dermopharmaceutiques selon l'une quelconque des revendications 1 à 5, **caractérisées en ce que** les composés sont utilisés sous forme de solution, de dispersion, d'émulsion, de pâte ou de poudre, individuellement ou en pré-mélange ou sont véhiculés individuellement ou en pré-mélange par des vecteurs comme les macro-, micro- ou nanocapsules, des liposomes ou des chylomicrons, des macro-, micro- ou nanoparticules, ou les microéponges, ou adsorbé sur des polymères organiques poudreux, les talcs, bentonites et autres supports minéraux.

7. Compositions cosmétiques ou dermopharmaceutiques selon l'une quelconque des revendications 1 à 6, **caractérisées en ce que** les composés sont utilisés individuellement ou en pré-mélange dans toute forme galénique à savoir lotions, laits ou crèmes émollients, laits ou crèmes pour les soins de la peau ou des cheveux, crèmes, lotions ou laits démaquillants, bases de fond de teint, lotions, laits ou crèmes anti-solaires, lotions, laits ou crèmes de bronzage artificiel, crèmes ou mousses de rasage, lotions après-rasage, shampooings, rouges à lèvres, mascaras ou vernis à ongles.

8. Compositions cosmétiques ou dermopharmaceutiques selon les revendications 1 à 7, **caractérisées en ce qu'**elles contiennent au moins un, préférentiellement plusieurs autres ingrédients habituellement utilisés en cosmétique dans un mélange cosmétiquement acceptable choisis parmi les catégories suivantes : solvants organiques ou hydroglycoliques, corps gras d'extraction ou de synthèse, épaississants ioniques ou non ioniques, adoucissants, opacifiants, stabilisants, émolliants, silicones, α-hydroxyacides, agents anti-mousse, agents hydratants, vitamines, parfums, conservateurs, séquestrants, colorants, polymères gélifiants et viscosants, tensioactifs et émulsifiants, autres principes actifs hydro- ou liposolubles, extraits végétaux, extraits tissulaires, extraits marins, filtres solaires, antioxydants.

9. Compositions selon la revendication 8, **caractérisées en ce qu'**elles contiennent un extrait de *glaucium flavum.*

10. Compositions selon la revendication 9, **caractérisées en ce que** la quantité de l'extrait de *glaucium flavum* incorporée dans les préparations cosmétiques ou dermopharmaceutiques est comprise entre 0,01 et 100 % (p/p), préférentiellement entre 0,1 et 10 % en poids de la composition totale finale.

11. Composé **caractérisé en ce qu'**il est la 2,9-diacétytoxy-1,10-diméthoxy-6-méthyl-noraporphine représentée par la formule **II** suivante :

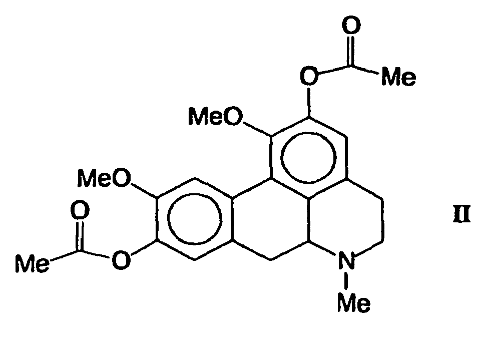

12. Composé selon la revendication 11, **caractérisé en ce qu'**il est obtenu à partir de toute source d'approvisionnement, en particulier par voie de synthèse chimique, ou par extraction végétale.

13. Utilisation du composé selon l'une quelconque des revendications 11 ou 12 ou des compositions définies à l'une quelconque des revendications 1 à 10 dans l'objectif d'une diminution de la pigmentation, en particulier pour l'éclaircissement du teint, l'atténuation des taches cutanées de sénescence, l'homogénéisation de la coloration de la peau, l'éclaircissement de toute pigmentation associée à la mélanine, y compris celle des cheveux.

14. Utilisation du composé selon l'une quelconque des revendications 11 ou 12 ou des compositions définies à l'une quelconque des revendications 1 à 10 pour une action cosmétique anti-âge, anti-vieillissement, antiradicalaire ou anti-oxydante.

15. Utilisation du composé selon l'une quelconque des revendications 11 ou 12 ou des compositions définies à l'une quelconque des revendications 1 à 10, dans l'objectif d'un amincissement, d'une réduction de la peau d'orange et/ou de la surcharge pondérale des hanches et des cuisses, du traitement de la cellulite, du raffermissement cutané, d'un amincissement de certaines parties du visage, plus généralement de l'inhibition de la lipogénèse.

16. Utilisation du composé selon l'une quelconque des revendications 11 ou 12 ou des compositions définies à l'une quelconque des revendications 1 à 10, sous une forme liée ou incorporée ou absorbée ou adsorbée à des macro-, micro- et nanoparticules, des macro-, micro- et nanocapsules, dans les textiles, fibres synthétiques ou naturelles, laines ou tous matériaux susceptibles d'être utilisés pour réaliser des vêtements et sous-vêtements de jour ou de nuit, directement au contact de la peau ou des cheveux pour en permettre une délivrance topique continue.

17. Utilisation du composé selon l'une quelconque des revendications 11 ou 12 ou des compositions définies à l'une quelconque des revendications 1 à 10, pour la préparation de médicaments destinés aux soins de la peau, particulièrement à son blanchissement et à sa moindre coloration lors d'expositions aux UV naturels ou artificiels, ou dans l'objectif d'un traitement anti-âge, anti-vieiNissement, antiradicalaire, antioxydant, ou encore dans l'objectif d'un traitement amincissant, d'une réduction de la peau d'orange et/ou de la surcharge pondérale des hanches et des cuisses, le traitement de la cellulite et le raffermissement cutané, plus généralement de l'inhibition de la lipogénèse.

## Claims

1. Cosmetic or dermopharmaceutical compositions comprising: one or several compounds having the general formula I: 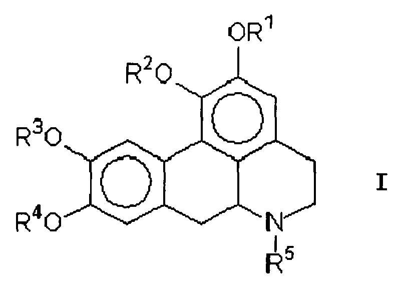 in which: the groups R¹, R², R³, R⁴ and R⁵, which may be the same or different, each one of them consists in a hydrogen atom, an alkyl, aryl, aralkyl, acyl, sulfonyl or sugar group, and the salts of those compounds with an acid that is acceptable in cosmetic terms, in the form of their optically pure isomer and any mixture of those isomers, alone or in association, and a cosmetically acceptable carrier.

2. Compositions according to claim 1 wherein said compound corresponds to 2, 9-diacetyloxy-1,10-dimethoxy-6-methyl-noraporphine (formula I where R¹ = R⁴ = COCH₃, R² = R³ = CH₃, R⁵ = CH₃) or the 1,2,9,10-tetramethoxy-6-methyl-noraporphine (formula I where R¹ = R² = R³ = R⁴ = R⁵ = CH₃).

3. Compositions according to claim 1 or 2, wherein said compounds are occurred from any source of supply, in particular by chemical synthesis, or by plant extraction.

4. Composition according to claim 3, wherein said compounds are obtained by plant extraction from glaucium flavum.

5. Cosmetic or dermopharmaceutical compositions according to any of claims 1 to 4, wherein said compounds are present at a concentration between 0.0001 (w/w) and 50% (w/w), preferably between 0,001 % (w/w) and 20 % (w/w).

6. Cosmetic or dermopharmaceutical compositions according to any of claims 1 to 5, wherein said compounds are used in the form of a solution, dispersion, emulsion, paste, or powder, individually or in the form of a premix, or are included individually or as a premix in vehicles constituted by carriers such as macro-, micro-, or nanocapsules, liposomes or chylomicrons, macro-, micro-, or nanoparticles or microsponges, or are adsorbed on organic polymer powders, talcs, bentonites, or other inorganic supports.

7. Cosmetic or dermopharmaceutical compositions according to any of claims 1 to 6, wherein said compounds are used individually or in the form of a premix in any formulation, namely emollient lotions, emollient milks or emollient creams, milks and creams for skin or hair care, make-up-removing cleansing creams, lotions or milks, foundation tint bases, sun-screen lotions, milks, or creams, artificial suntan lotions, milks, or creams, shaving creams, shaving foams, aftershave lotions, shampoos, lipsticks, mascaras, or nail varnishes.

8. Cosmetic or dermopharmaceutical compositions according to any of claims 1 to 7, **characterized in that** they contain at least one and preferably several other ingredients commonly used in cosmetic practice in a cosmetically acceptable mixture, selected from among the following categories: organic or hydroglycolic solvents, fatty substances obtained by extraction or synthesis, ionic or non-ionic thickeners, softeners, opacifiers, stabilizers, emollients, silicones, α-hydroxy acids, antifoaming agents, moisturizing agents, vitamins, perfumes, preservatives, sequestrating agents, colouring agents, gel-forming and viscosity-increasing polymers, surfactants and emulsifiers, other water- or fat-soluble active principles, plant extracts, tissue extracts, marine extracts, sun filters, and antioxidants

9. Compositions according to claim 8 containing a glaucium flavum extract.

10. Compositions according to claim 9, wherein said glaucium flavum extract is incorporated in cosmetic or dermopharmaceutical preparations at concentrations varying between 0.01 and 100 % (w/w), preferably between 0.1 and 10 % in weight of the final total composition.

11. Compound **characterized in that** it corresponds to 2, 9-diacetyloxy-1, 10-dimethoxy-6-methyl-noraporphine, hereafter represented by the formula II:

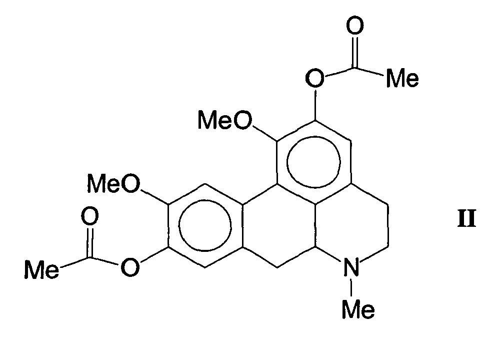

12. Compound according to claim 11, wherein said compound is occurred from any source of supply, in particular by chemical synthesis, or by plant extraction.

13. Use of compound according to any of claims 11 or 12, or compositions described in any of above claims 1 to 10 with the aim of decreasing pigmentation, in particular to lighten the complexion, attenuate senile lentigo, homogenize skin color, or lighten any pigmentation associated with melanin including that of the hair.

14. Use of compound according to any of claims 11 or 12, or compositions described in any of claims 1 to 10 with the aim of an anti-age, anti-ageing, antiradicalaire, antioxydant action.

15. Use of compound according to any of claims 11 or 12, or compositions described in any of claims 1 to 10, with the aim of a slimming, a reduction of the orange peel and/or overloads of the thighs and the hips, anti-cellulite treatment, skin firming, to refine contours of the face, more generally the lipogenesis inhibition.

16. Use of compound according to any of claims 11 or 12, or compositions described in any of claims 1 to 10, in a form bound to or incorporated in or absorbed in or adsorbed on macro-, micro-, and nanoparticles, or macro-, micro-, and nanocapsules, for the treatment of textiles, natural or synthetic fibres, wools, and any materials that may be used for clothing or underwear for day or night intended to come into contact with the skin or hair to exert their cosmetic effect via this skin/textile contact and to permit continuous topical delivery.

17. Use of compound according to any of claims 11 or 12, or compositions described in any of claims 1 to 10, for the preparation of medicinal products intended for skin care, particularly skin lightening and reducing its coloration under exposure to natural or artificial UV radiation, or in the objective of an anti-age, anti-ageing, antiradicalaire, antioxydant treatment, or in the objective of a slimming treatment, a reduction of the orange peel and/or overloads of the thighs and the hips, a anti-cellulite treatment, skin firming, more generally the lipogenesis inhibition.

## Patentansprüche

1. Kosmetische und dermopharmazeutische Zusammensetzungen, die eine oder mehrere Verbindungen gemäß Formel I umfassen: 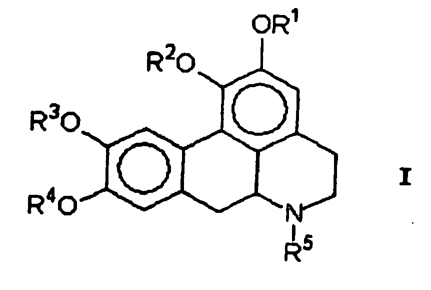 wobei:
die Gruppen R¹, R², R³, R⁴ und R⁵, die identisch oder unterschiedlich sein können, jede ein Wasserstoffatom, eine Alkyl-, Aryl-, Aralkyl-, Acyl-, Sulfonyl- oder Zuckergruppe darstellen,
und ihr Salz mit einer kosmetisch akzeptablen Säure, in Form ihres reinen optischen Isomers oder einer Mischung der Isomere,
allein oder in Kombination und eine kosmetisch akzeptable Grundmasse.

2. Zusammensetzungen gemäß Anspruch 1, **dadurch gekennzeichnet , dass** die Zusammensetzung 2,9-Diacetyloxy-1,10-dimethoxy-6-methylnoraporphin (Formel 1 mit R¹ = R⁴ = COCH₃, R² = R³ = CH₃, R⁵ = CH₃) oder 1,2,9,10-Tetramethoxy-6-methylnoraporphin (Formel I mit R¹ = R² = R³ = R⁴ = R⁵ = CH₃) ist.

3. Zusammensetzungen gemäß Anspruch 1 oder 2, **dadurch gekennzeichnet , dass** die Verbindungen aus allen Beschaffungsquellen erhalten werden, insbesondere durch chemische Synthese oder durch pflanzliche Extraktion.

4. Zusammensetzungen gemäß Anspruch 3, **dadurch gekennzeichnet , dass** die Verbindungen durch pflanzliche Extraktion aus *glaucium flavum* erhalten werden.

5. Kosmetische oder dermopharmazeutische Zusammensetzungen gemäß einem der Ansprüche 1 bis 4, **dadurch gekennzeichnet , dass** die Verbindungen in Konzentrationen verwendet werden, die zwischen 0,0001 % (m/m) und 50 % (m/m), bevorzugt zwischen 0,001 % (m/m) und 20 % (m/m) verwendet werden.

6. Kosmetische oder dermopharmazeutische Zusammensetzungen gemäß einem der Ansprüche 1 bis 5, **dadurch gekennzeichnet , dass** die Verbindungen in Form einer Lösung, Dispersion, Emulsion, Paste oder eines Puders verwendet werden, einzeln oder als Vormischung oder einzeln oder in Formmischung durch Träger wie Makro-, Mikro- oder Nanokapseln, Liposomen oder Chylomikronen, Makro-, Mikro- oder Nanopartikel oder Mikroschwämme oder adsorbiert auf pulverförmigen organischen Polymeren, Talkum, Bentoniten oder anderen mineralischen Trägem transportiert werden.

7. Kosmetische oder dermopharmazeutische Zusammensetzungen gemäß einem der Ansprüche 1 bis 6, **dadurch gekennzeichnet , dass** die Verbindungen einzeln oder in Vormischung in allen galenischen Formen, und zwar erweichenden Lotionen, Milch oder Cremes, Milch oder Cremes für die Haut- oder Haarpflege, Reinigungscremes, -lotionen oder -milch, Makeup-Grundlagen, Sonnenschutzlotionen, -milch oder -cremes, Bräunungslotionen, -milch oder -cremes, Rasiercremes oder -schäume, Aftershave-Lotionen, Shampoos, Lippenstifte, Wimperntuschen oder Nagellack, verwendet werden.

8. Kosmetische oder dermopharmazeutische Zusammensetzungen gemäß einem der Ansprüche 1 bis 7, **dadurch gekennzeichnet , dass** sie mindestens einen, bevorzugt mehrere Bestandteile umfassen, die üblicherweise in einer Kosmetik in einer kosmetisch akzeptablen Mischung verwendet werden, ausgewählt aus den folgenden Gruppen: organische oder hydroglycolische Lösungsmittel, extrahierte oder synthetische Fette, ionische oder nicht ionische Verdickungsmittel, Reizlinderungsmittel, Trübungsmittel, Stabilisatoren, Weichmacher, Silikone, α-Hydroxysäuren, Antischaummittel, Feuchtigkeitsspender, Vitamine, Parfüme, Konservierungsmittel, Komplexbildner, Farbstoffe, polymere Geliermittel und Viskositätsregler, Tenside und Emulgatoren, andere wasser- oder fettlösliche Wirkstoffe, pflanzliche Extrakte, Gewebeextrakte, Meeresextrakte, Sonnenfilter, Antioxidationsmittel.

9. Zusammensetzungen gemäß Anspruch 8, **dadurch gekennzeichnet , dass** sie einen Extrakt aus *glaucium flavum* enthalten.

10. Zusammensetzungen gemäß Anspruch 9, **dadurch gekennzeichnet , dass** die Menge des enthaltenen Extrakts aus *glaucium flavum* in den kosmetischen oder dermopharmazeutischen Zubereitungen zwischen 0,01 und 100 % (m/m), bevorzugt zwischen 0,1 und 10 % des Gesamtgewichts der Endzusammensetzung umfasst.

11. Verbindung, **dadurch gekennzeichnet , dass** sie 2,9-Diacetyloxy-1,10-dimethoxy-6-methylnoraporphin gemäß der folgenden Formel II ist:

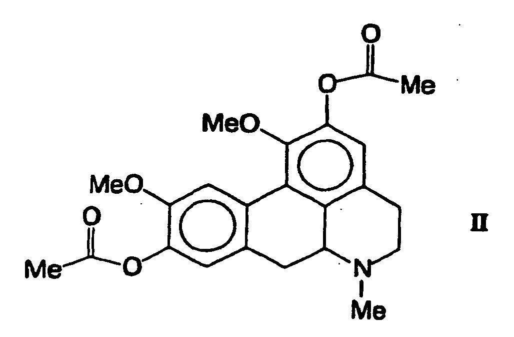

12. Verbindung gemäß Anspruch 11, **dadurch gekennzeichnet , dass** sie durch alle Beschaffungsquellen erhalten wird, insbesondere durch chemische Synthese oder durch pflanzliche Extraktion.

13. Verwendung der Verbindung gemäß einem der Ansprüche 11 oder 12 oder der Zusammensetzungen, die in einem der Ansprüche 1 bis 10 definiert sind, mit dem Ziel einer Verringerung der Pigmentation, insbesondere für die Aufhellung der Gesichtsfarbe, die Abschwächung von Altersflecken, die Homogenisierung der Hautfarbe, die Aufhellung aller Pigmentation, die mit Melanin verbunden ist, einschließlich jener der Haare.

14. Verwendung der Verbindung gemäß einem der Ansprüche 11 oder 12 oder der Zusammensetzungen, die in einem der Ansprüche 1 bis 10 definiert sind, für eine kosmetische Antialter-, Antialterungs-, antiradikalische oder Antioxidationswirkung.

15. Verwendung der Verbindung gemäß einem der Ansprüche 11 oder 12 oder der Zusammensetzungen, die in einem der Ansprüche 1 bis 10 definiert sind, mit dem Ziel des Abnehmens, einer Verringerung der Orangenhaut und/oder des Übergewichts der Hüften und der Schenkel, der Behandlung der Cellulite, der Hautstraffung; der Verschlankung bestimmter Bereiche des Gesichts, allgemeiner der Hemmung der Lipogenese.

16. Verwendung der Verbindung gemäß einem der Ansprüche 11 oder 12 oder der Zusammensetzungen, die in einem der Ansprüche 1 bis 10 definiert sind, in einer Form, die gebunden an, eingeschlossen in, absorbiert in oder absorbiert an Makro-, Mikro- und Nanopartikel, Makro-, Mikro- und Nanokapseln, in Textilien, synthetischen oder natürlichen Fasern, Wolle oder alle Materialien, die geeignet sind, für die Herstellung von Kleidung und Unterbekleidung für den Tag oder die Nacht verwendet zu werden, die in direktem Kontakt mit der Haut oder dem Haar ist, um eine kontinuierliche örtliche Freisetzung zu erlauben, ist.

17. Verwendung der Verbindung gemäß einem der Ansprüche 11 oder 12 oder der Zusammensetzungen, die in einem der Ansprüche 1 bis 10 definiert sind, für die Herstellung von Medikamenten, die für die Hautpflege bestimmt sind, insbesondere deren Bleichung und die Verringerung der Färbung bei der Exposition gegenüber natürlichem oder künstlichem UV, oder mit dem Ziel einer Antialter-, Antialterungs-, antiradikalischen, Antioxidationsbehandlung oder auch mit dem Ziel einer Schlankheitsbehandlung, einer Verringerung der Orangenhaut und/oder des Übergewichts der Hüften und der Schenkel, einer Behandlung der Cellulite und einer Hautstraffung, allgemeiner einer Hemmung der Lipogenese.
